Europäisches Patentamt

European Patent Office

Office européen des brevets

(19)

(11) Veröffentlichungsnummer : **0 061 014**
**B1**

# EUROPÄISCHE PATENTSCHRIFT

(12)

(45) Veröffentlichungstag der Patentschrift :
30.01.85

(51) Int. Cl.⁴ : **A 61 B   5/08**

(21) Anmeldenummer : 82101465.1

(22) Anmeldetag : 26.02.82

(54) **Atemmonitor.**

(30) Priorität : 20.03.81 DE 3110843

(43) Veröffentlichungstag der Anmeldung :
29.09.82 Patentblatt 82/39

(45) Bekanntmachung des Hinweises auf die Patenterteilung : 30.01.85 Patentblatt 85/05

(84) Benannte Vertragsstaaten :
DE FR GB

(56) Entgegenhaltungen :
DE-A- 2 527 475
FR-A- 1 575 190
FR-A- 2 267 734

(73) Patentinhaber : **Hewlett-Packard GmbH**
**Herrenberger Strasse 110**
**D-7030 Böblingen (DE)**

(72) Erfinder : **Pross, Gerhard, Dipl.-Ing.**
**Breitensteiner Weg 11**
**D-7031 Weil Im Schönbuch (DE)**
Erfinder : **Rochlitzer, Frank**
**Kieferweg 9**
**D-7031 Altdorf (DE)**

(74) Vertreter : **Schulte, Knud, Dipl.-Ing.**
**c/o Hewlett-Packard GmbH Europ. Patent- und Li-**
**zenzabteilung Postfach 1430 Herrenberger Strasse**
**130 D-7030 Böblingen (DE)**

Jouve, 18, rue St-Denis, 75001 Paris, France

## Beschreibung

Die Erfindung betrifft einen Atemmonitor gemäß dem Oberbegriff des Anspruchs 1.

Derartige Atemmonitoren werden zum Überwachen der Atmung gefährdeter Patienten beispielsweise in Intensivstationen oder bei Frühgeburten verwendet. Sie zeichnen im allgemeinen die Atemtätigkeit auf und geben bei Atemstillstand (Apnoe) ein Alarmsignal ab, wenn die Dauer des Atemstillstandes einen vorgegebenen Wert überschreitet. Die Atemtätigkeit wird meistens durch Messen der Thoraximpedanz des Patienten überwacht. Es sind jedoch auch andere Meßmethoden zum Erfassen der Atemaktivität bekannt, beispielsweise Messen von Temperaturänderungen der Atemluft oder Änderungen des Auflagedrucks des Körpers auf der Unterlage.

Da die Amplitude der Atemsignale von Patient zu Patient verschieden ist und sich auch bei demselben Patienten mit der Zeit ändern kann, ist bei einigen bekannten Atemmonitoren ein selbsttätiges Anpassen der Empfindlichkeit entsprechend der Amplitude der Atemsignale vorgesehen (siehe z. B. FR-A-2 267 734). Dieses Anpassen erfolgt mit einer gewissen Verzögerung entsprechend einer vorgegebenen Zeitkonstante. Um dabei zu verhindern, daß Rausch- und andere Störsignale fälschlich als Atemsignale kleiner Amplitude gedeutet werden, ist eine Mindest-Amplitudenschwelle vorgesehen, bei deren Unterschreitung keine effektive Atmung mehr erkannt wird. Wird diese Amplitudenschwelle für einen längeren Zeitraum unterschritten, wird bei Erreichen der zeitlichen Alarmgrenze ein Alarm ausgelöst.

Es kann vorkommen, daß Störsignale diese Mindest-Amplitudenschwelle übersteigen und fälschlich als effektive Atemsignale gedeutet werden, obwohl ein Atemstillstand vorliegt. Solche Störsignale können vor allem von der Herztätigkeit herrühren. In FR-A-2 267 734 ist ein Atemmonitor beschrieben, der auch solche Störsignale unterdrückt.

Die vorliegende Erfindung löst demgegenüber durch das Kennzeichen von Anspruch 1 die Aufgabe, einen Atemmonitor gemäß dem Oberbegriff derart weiterzubilden, daß nicht nur ein völliger Atemstillstand sondern auch eine unzureichende Atmung erkannt wird.

Der Erfindung liegt die Erkenntnis zugrunde daß zwei voneinander abhängige Voraussetzungen erfüllt sein müssen, um einen für den Patienten ausreichenden Gasaustausch sicherzustellen. Eine effektive Atmung ist sowohl mit relativ langsamen und tiefen Atemzügen als auch mit relativ schnellen und flacheren Atemzügen möglich. Wird beispielsweise langsam geatmet, so muß eine bestimmte Mindestatemtiefe erreicht werden, um einen ausreichenden Gasaustausch in der Lunge durch entsprechende Lungenbelüftung (Ventilation) zu erreichen. Erfindungsgemäß wird daher die Amplitudenschwelle zunächst gleitend bis auf den genannten Mindestwert abgesenkt, solange die Atemfrequenz über einer gewissen Mindestfrequenz liegt. Hält eine Atempause allerdings länger an, so wird die Amplitudenschwelle gleitend angehoben, da eine länger an, so wird die Amplitudenschwelle gleitend angehoben, da eine längere Atempause eine Verringerung der Atemfrequenz bedeutet. Treten nun bei angehobener Amplitudenschwelle unzureichende, flache Atemzüge oder Störsignale auf, so werden diese nicht erfaßt. Dieser Zustand wird also wie ein Atemstillstand behandelt. Erst ein tieferer Atemzug, dessen Signalamplitude gemäß einer Weiterbildung der Erfindung mit zunehmendem Zeitlichen Abstand vom letzten erfaßten Atemzug noch zunehmen muß, wird wieder als effektiver Atemzug betrachtet und verhindert den Alarm. Nach dem Auftreten eines effektiven Atemzuges wird die Amplitudenschwelle wieder gleitend abgesenkt.

Gemäß einer Weiterbildung der Erfindung kann auch bei höheren Atemfrequenzen die Amplitudenschwelle angehoben werden, um sicherzustellen, daß auch dort effektiv geatmet und nicht nur « Totvolumen » hin- und herbewegt wird.

Vorteilhafte Ausführungsformen der Erfindung sind in den übrigen Unteransprüchen gekennzeichnet.

Die Erfindung wird im folgenden anhand eines Ausführungsbeispiels in Verbindung mit der zugehörigen Zeichnung erläutert, in der ein Blockschaltbild eines erfindungsgemäßen Atemmonitors dargestellt ist.

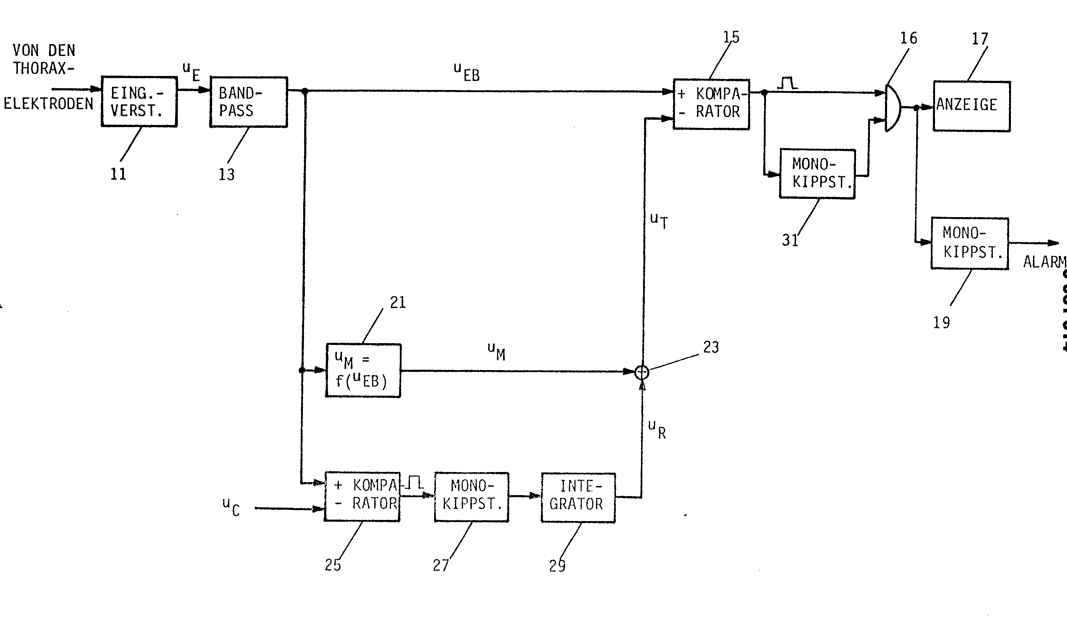

In der Zeichnung nicht dargestellte Thoraxelektroden stehen mit einem üblichen Eingangsverstärker 11 in Verbindung, der im vorliegenden Beispiel eine definierte Signalspannung von 2,35 mV/$\Delta$ 1 $\Omega$ abgibt. Diese mit uE bezeichnete Signalspannung ist also proportional zur Änderung der Thoraximpedanz. Die Spannung uE wird zunächst einem Bandpaßfilter 13 zugeführt, welches im vorliegenden Beispiel eine untere Grenzfrequenz von 0,2 Hz (12/min) und eine obere Grenzfrequenz von 2 Hz (120/min) aufweist. Die Bandpaßcharakteristik bewirkt, daß Signale mit Frequenzen außerhalb der Grenzfrequenzen gedämpft werden, da sie für die Überwachung der Atemtätigkeit nicht relevant sind. Das Bandpaßfilter 13 hat zusätzlich einen Verstärkungsfaktor von 426, so daß seine Ausgangsspannung uEB im Durchlaßbereich 1 V/$\Delta$ 1 $\Omega$ beträgt.

Die Ausgangssignalspannung uEB des Bandpaßfilters 13 wird dem positiven Eingang eines Komparators 15 zugeführt, dessen negativem Eingang die Amplitudenschwelle uT zugeführt wird, deren Erzeugung weiter unten erläutert wird. Der Komparator 15 gibt immer dann einen Ausgangsimpuls ab, wenn die Spitze/Spitze-Amplitude der Spannung uEB größer als die Amplitudenschwelle uT ist. Diese Impulse zeigen jeweils einen effektiven Atemzug an und können z. B. in einer Anzeigeeinheit 17 weiterverarbeitet werden. Außerdem werden sie einer retriggerbaren monostabilen Kippstufe 19 zugeführt, deren Verzögerungszeit einer vorgegebenen Alarmgrenze entspricht. Ist der Impulsabstand kürzer als die Alarmgrenze, z. B. 30 s, so bleibt das Ausgangssignal der monostabilen Kippstufe 19 auf seinem durch das Eingangssignal angeregten Zustand stehen. Dieser Zustand bedeutet, daß die Atmung effektiv ist. Wird jedoch die Alarmgrenze überschritten, kippt die monostabile Kippstufe 19 in ihren Grundzustand zurück, und dies wird als Alarm angezeigt oder auf andere Weise deutlich gemacht. Die Ausgangsimpulse des Komparators 15 werden jedoch nur zur Anzeigeeinheit 17 bzw. zur monostabilen Kippstufe 19 durchgelassen, wenn sie von einem UND-Glied 16 durchgelassen werden, wie weiter unten beschrieben ist.

Die Amplitudenschwelle uT wird in einem Summierglied 23 als Summe zweier Schwellenspannungen uM und uR gebildet. Die Schwellenspannung uM liefert ein Schwellenregler 21. Der Schwellenregler 21 erzeugt die Schwellenspannung uM als bestimmten Bruchteil, z. B. 60 %, der Spitze/Spitze-Amplitude der gefilterten Signalspannung uEB. Der Schwellenregler 21 ist als solcher bekannt und kann z. B. zusätzlich die Schwellenspannung uM anheben, wenn die Frequenz der Signalspannung uEB gleich der zusätzlich gemessenen Herzfrequenz des Patienten ist, wie in FR-A-22 67 734 beschrieben. Im vorliegenden Beispiel liefert der Schwellenregler eine Mindest-Schwellenspannung uM min = 100 mV.

Die gefilterte Signalspannung uEB wird weiterhin dem positiven Eingang eines Komparators 25 zugeführt, dessen negativem Eingang eine feste Schwellenspannung uC zugeführt wird, die im vorliegenden Beispiel 80 mV beträgt, was einer Thoraximpedanzänderung von 80 mΩ entspricht. Dies ist die vorgegebene absolute Untergrenze für die Weiterverarbeitung der Atemsignale. Der Komparator 25 gibt nur dann einen Ausgangsimpuls ab, wenn die Spitze/Spitze-Amplitude von uEB größer als uC ist.

Die Ausgangsimpulse des Komparators 25 werden einer retriggerbaren monostabilen Kippstufe 27 zugeführt. Die Verzögerungszeit der monostabilen Kippstufe 27 beträgt im vorliegenden Beispiel 2,2 Sekunden. Dies ist die Periodendauer bei einer Atemfrequenz von etwa 27/min. Das Ausgangssignal der monostabilen Kippstufe 27 bleibt solange Null, wie der Komparator 25 seine Impulse in einen Mindestabstand von 2,2 Sekunden abgibt. Wird dieser Impulsabstand überschritten, so kippt das Ausgangssignal der monostabilen Kippstufe 27 für die Dauer der Zeitüberschreitung auf einen positiven Wert.

Das Ausgangssignal der monostabilen Kippstufe 27 wird einem Integrator 29 zugeführt. Dessen Ausgangsspannung uR bleibt 80 mV (entsprechend uC) oder sinkt mit einer bestimmten Absinkrate auf 80 mV, wenn sein Eingangssignal Null ist. Positive Ausgangssignale der monostabilen Kippstufe 27 lassen jedoch die Spannung uR mit einer bestimmten Anstiegrate ansteigen, und zwar um so höher, desto länger das positive Ausgangssignal der monostabilen Kippstufe 27 ist, d. h. desto länger die Verzögerungszeit von 2,2 Sekunden übereschritten wurde. Die Ausgangsspannung uR des Integrators ist nach oben auf 1,8 V begrenzt. Die Anstiegrate beträgt 180 mV/s und die Absinkrate beträgt 450 mV/s.

Da die Spannung uR als Summand in die Amplitudenschwelle uT eingeht, wird auch die Ansprechschwelle des Komparators 15 entsprechend erhöht, wenn die Spannung uR ansteigt. Je höher aber die Amplitudenschwelle uT ansteigt, desto größer muß die Spitze/Spitze-Amplitude von uEB sein, d. h. desto tiefer muß der jeweilige Atemzug sein, damit der Komparator 15 noch einen Ausgangsimpuls abgibt. Dadurch wird verhindert, daß z. B. nach einem Atemstillstand eine flache, ineffektive Atmung oder ein Störsignal durch Muskelbewegung bereits ausreicht, um die Auslösung eines Alarms zu verhindern.

Im bisher beschriebenen Ausführungsbeispiel wird von dem Schwellenregler 21 einmal abgesehen, die Amplitudenschwellen-Charakteristik im wesentlichen durch zwei von der Atemfrequenz abhängige Schaltungen bestimmt, nämlich einerseits das Bandpaßfilter 13 und andererseits den aus Komparator 25, monostabiler Kippstufe 27, Integrator 29 und Summierglied 23 bestehenden Pfad für das Beeinflussen der Amplitudenschwelle.

Abgesehen von der Filtercharakteristik des Bandpaßfilters 13 ist bei der vorbeschriebenen Überwachung keine obere Atemfrequenzgrenze vorhanden. Mittels einer retriggerbaren monostabilen Kippstufe 31 läßt sich erreichen, daß oberhalb einer Höchstfrequenz von z. B. 200/min die Ausgangssignale des Komparators 15 unterdrückt werden, da solche Signale mit Sicherheit keine effektive Atmung mehr anzeigen. Die monostabile Kippstufe 31 wird von der abfallenden Flanke jedes Ausgangsimpulses des Komparators 15 getriggert. Ist der Impulsabstand des Ausgangssignals des Komparators 15 kleiner als 300 ms, so ist das Ausgangssignal der monostabilen Kippstufe 31 ständig Null. Das UND-Glied 16 bleibt dann gesperrt. Ist der Impulsabstand jedoch größer als 300 ms, geht das Ausgangssignal der monostabilen Kippstufe 31 auf logisch Eins, bevor der Komparator 156 den nächstfolgenden Impuls abgibt, so daß dieser das UND-Glied 16 passieren kann.

**Ansprüche**

1. Atemmonitor, bei welchem die Atemtätigeit eines Patienten durch Messen von Atemsignalen, vorzugsweise von atmungsbedingten Änderungen der Thoraximpedanz, überwacht wird, mit einem Komparator (15), der Atemsignale unterhalb einer bestimmten Amplitudenschwelle (uT) unterdrückt und mit einem Schwellenregler (21), der die Amplitudenschwelle (uT) mit kleiner werdender Amplitude der Atemsignale gleitend bis auf einen Mindestwert herabsetzt, gekennzeichnet durch einen mit dem ersten Schwellenregler zusammenwirkenden zweiten Schwellenregler (23, 25, 27, 29), der die Amplitudenschwelle (uT) gleitend auf einen höheren Wert anhebt, wenn die Frequenz der Atemsignale unter eine vorgegebene Mindestfrequenz absinkt, und der die Amplitudenschwelle (uT) gleitend wieder bis auf den ursprünglichen Wert herabsetzt, wenn die Frequenz der Atemsignale wieder über die vorgegebene Mindestfrequenz ansteigt.

2. Atemmonitor nach Anspruch 1, gekennzeichnet durch einen Atem-Höchstfrequenzdetektor (16, 31), der oberhalb einer vorgegebenen Höchstfrequenz die erkannten Atemsignale vollständig unterdrückt.

3. Atemmonitor nach Anspruch 1 oder 2, gekennzeichnet durch einen Integrator (29), der während der Zeitintervalle, in denen die der vorgegebenen Mindestfrequenz entsprechende Periodendauer überschritten wird, die Amplitudenschwelle mit einer bestimmten Anstiegrate anhebt und in den verbleibenden Zeitintervallen die Amplitudenschwelle mit einer bestimmten Absinkrate absenkt.

4. Atemmonitor nach Anspruch 3, dadurch gekennzeichnet, daß die Anstiegrate etwa einer Änderung um den Mindestwert der Amplitudenschwelle pro Sekunde entspricht und daß die Absinkrate etwa einer Änderung um das 2,5-fache des Mindestwertes der Amplitudenschwelle pro Sekunde entspricht.

## Claims

1. A respiration monitor wherein the respiration of a patient is monitored by measurement of respiratory signals, preferably of changes of the thorax impedance caused by respiration, with a comparator (15) which suppresses respiratory signals below a certain amplitude threshold (uT) and with a threshold regulator (21) which in response to a decreasing amplitude of the respiratory signals glidingly reduces the amplitude threshold (uT) to a minimum value, characterized by a second threshold regulator (23, 25, 27, 29) acting in combination with the first threshold regulator, which glidingly raises the amplitude threshold (uT) to a higher value when the frequency of the respiratory signals falls beneath a predetermined minimum frequency, and which again glidingly reduces the amplitude threshold (uT) to its original value when the frequency of the respiratory signals rises above the predetermined minimum frequency again.

2. A respiration monitor as in claim 1, characterized by a peak respiration frequency detector (16, 31) which completely suppresses the detected respiration signals above a predetermined peak frequency.

3. A respiration monitor as in claims 1 or 2, characterized by an integrator (29) which raises the amplitude threshold with a certain rate of rise during the time intervals in which the period duration corresponding to the predetermined minimum frequency is exceeded, and which decreases the amplitude threshold with a certain rate of descent in the remaining time intervals.

4. Respiration monitor as in claim 3, characterized in that the rate of rise corresponds approximately to a change by the minimum value of the amplitude threshold per second, and that the rate of descent corresponds approximately to a change by 2.5 times the minimum value of the amplitude threshold per second.

## Revendications

1. Dispositif de surveillance de la respiration, par lequel est surveillée l'activité respiratoire d'un patient par la mesure de signaux respiratoires, de préférence par des variations d'impédance du thorax conditionnées par la respiration, avec un comparateur (15), qui réprime les signaux respiratoires inférieurs à un seuil déterminé d'amplitude (uT) et avec un régulateur de seuil (21), qui abaisse le seuil d'amplitude (uT) avec une amplitude des signaux respiratoires diminuant en glissant jusqu'à une valeur minimale caractérisé par un second régulateur de seuil (23, 25, 27, 29) coopérant avec le premier régulateur de seuil, qui élève en glissant le seuil d'amplitude (uT) à une valeur supérieure, quand la fréquence des signaux respiratoires décroît au-dessous d'une fréquence minimale prédéterminée, et qui abaisse à nouveau en glissant le seuil d'amplitude (uT) jusqu'à la valeur originelle, quand la fréquence des signaux respiratoires croît à nouveau au-delà de la fréquence minimale prédéterminée.

2. Dispositif de surveillance selon la revendication 1, caractérisé par un détecteur à fréquence maximale de respiration (16, 31), qui réprime complètement les signaux respiratoires reconnus au-delà d'une fréquence maximale prédéterminée.

3. Dispositif de surveillance selon la revendication 1 ou 2, caractérisé par un intégrateur (29) qui, pendant l'intervalle de temps dans lequel est franchie la durée de période correspondant à la fréquence minimale prédéterminée, élève le seuil d'amplitude avec une vitesse de montée déterminée et qui, dans les intervalles de temps restants, fait décroître le seuil d'amplitude avec une vitesse de descente déterminée.

4. Dispositif de surveillance selon la revendication 3, caractérisé en ce que la vitesse de montée correspond à peu près à une variation de la valeur minimale du seuil d'amplitude par seconde, et en ce que la vitesse de descente correspond à peu près à une variation de 2,5 fois la valeur minimale du seuil d'amplitude par seconde.

0 061 014

VON DEN THORAX-ELEKTRODEN

$u_E$ — EING.-VERST. (11) — BAND-PASS (13) — $u_{EB}$

ANZEIGE (17)

MONO-KIPPST. (19) — ALARM

16

KOMPA-RATOR (15) $+$ $-$

MONO-KIPPST. (31)

$u_T$

23

$u_R$

INTE-GRATOR (29)

$u_M$

$u_M = f(u_{EB})$ (21)

MONO-KIPPST. (27)

KOMPA-RATOR (25) $+$ $-$

$u_C$

1